# EUROPEAN PATENT APPLICATION

(11) **EP 3 588 512 A1**
(43) Date of publication of application: **01.01.2020**
(21) Application number: 18179755.6
(22) Date of filing: 26.06.2018
(51) Int. Cl.: G16H 50/20, G06F 17/27

(54) **A COMPUTER-IMPLEMENTED METHOD, AN APPARATUS AND A COMPUTER PROGRAM PRODUCT FOR ASSESSING THE HEALTH OF A SUBJECT**

(71) Applicant: Koninklijke Philips N.V., 5656 AE Eindhoven (NL)
(72) Inventor: SHRUBSOLE, Paul Anthony, 5656 AE Eindhoven (NL); SARTOR, Francesco, 5656 AE Eindhoven (NL)
(74) Representative: de Haan, Poul Erik

(57) **Abstract**

According to an aspect, there is provided an apparatus (4, 6, 7) for assessing the health of a subject. The apparatus (4, 6, 7) comprises one or more processing units (8, 28, 48) configured to receive an input from a subject as part of a conversation between the subject and a virtual assistant module; analyse the received input to identify one or more terms relating to a medical condition or a potential medical condition of the subject; obtain physiological characteristic and/or activity measurements for the subject from one or more physiological characteristic and/or activity sensors (18) associated with the subject; process the identified one or more terms, medical history information for the subject and the obtained physiological characteristic and/or activity measurements to determine an adjustment to a measurement frequency and/or measurement sensitivity of the one or more physiological characteristic and/or activity sensors (18) in order to obtain additional context information for the subject, the additional context information comprising one or more additional physiological characteristic and/or activity measurements for the subject; adjust the measurement frequency and/or measurement sensitivity of the one or more physiological characteristic and/or activity sensors (18) according to the determined adjustment and obtain the additional context information using the one or more physiological characteristic and/or activity sensors (18); determine if a notification to a care provider is required based on the identified one or more terms, the medical history information, the obtained physiological characteristic and/or activity measurements and the obtained additional context information; generate, if a notification to a care provider is required, a summary for the care provider including information extracted from the conversation, the physiological characteristic and/or activity measurements and the additional context information; and cause the presentation of the generated summary to the care provider.

## Description

### FIELD OF THE INVENTION

The disclosure relates to a computer-implemented method, an apparatus and a computer program product for assessing the health of a subject.

### BACKGROUND OF THE INVENTION

So-called 'chat bots' or 'virtual assistants' are a new kind of user interface which utilize artificial intelligence (AI) to answer queries, talk, and help users in a way that mirrors rational discussion with another person. Chat bots are now being specialized for healthcare, and they can provide a much more natural interface to users when compared to entering data into web forms or traditional computer or smartphone applications (apps). The chat bots can act as a gateway between the healthcare provider and the patient (user) and can help to narrow down and identify the patient's problem before a consultation with a real doctor. Consequently, significant cost savings are expected through the use of chat bots.

The key technologies that will enable such healthcare chat bots are natural language processing, classifier technologies and knowledge systems based on clinical practice. Several chat bots are currently available. Your MD is a chatbot app that uses artificial intelligence to guide users to the most relevant health information, and is intended to replace the assistant of the general practitioner (GP). Lifelink is a conversational workflow engine for healthcare. Sensely delivers a practical nurse, telemedicine, patient education videos, monitoring of vital signs, photo capture, reminders, and contact information.

Patients who are treated for coronary artery disease are typically discharged from hospital very soon after their treatment (for percutaneous coronary intervention (PCI), discharge can be on the same day). Most modern healthcare systems offer a post-discharge care plan that involves medication titration, cardiac rehabilitation therapy, including physical exercise and education, and regular consultations that reduce in frequency over time. There is a heavy reliance on patient self-management and patient engagement to change lifestyle habits (e.g. physical activity, smoking and diet) and improve knowledge about their condition and medication.

Since coronary artery disease is so prevalent globally (and thus costly), clinicians are seeking solutions that improve the mid-to-long term outcomes of their patients (including quality of life) compared to current practice.

A connected care system has been developed for this purpose. A patient app provides activation tools to learn, monitor, motivate (through a coaching program) and contextualize their condition (in particular, their symptoms). In a particular example, the patient app can include a medication log, a symptoms log, a vital signs/physiological characteristic log (e.g. heart rate, blood pressure, activity level, weight, etc.), a chat and/or video call module, a care manager module that can provide the coaching to the patient, ask the patient questions, educate the patient and issue alerts, a heart rate zone manager and an activity management and planner module. A clinician app (used by cardiology nurses and rehabilitation (rehab) nurses) tracks patient progress prior to consultations and can be used to modify the care plan accordingly. In a particular example, the clinician app can include a clinical decision support module, a medication titration/adherence module, a symptoms management module, a care plan management module, a patient calibration module and a patient rehab management module. The frequency of usage per patient of the clinician app is much lower compared with tele-monitoring.

The time taken by a nurse to review data collected for each patient during their care plan is expected to be significantly lower when compared to high-acuity patients (such as heart failure patients). The problem then is how to keep a nurse or other healthcare practitioner sufficiently informed about the status of their post-discharge patients and prioritize the patients most in need, whilst keeping the patient engaged and effective in communicating their needs. For example, any symptoms experienced by the patient are difficult to diagnose without sufficient context information, such as an indication of what the patient was doing when or before the symptoms occurred, an indication of whether the symptoms are linked to their medication, an indication of how severe the symptoms were, etc. It is not feasible for the healthcare practitioner to review every symptom that is registered by every patient.

Therefore, there is a need for improvements in the collection of data for a patient to enable interpretation of symptoms, and improvements in the presentation of that data to a healthcare professional so that the healthcare professional can make the appropriate decision for the patient.

### SUMMARY OF THE INVENTION

According to a first specific aspect, there is provided an apparatus for assessing the health of a subject, the apparatus comprising one or more processing units configured to: receive an input from a subject as part of a conversation between the subject and a virtual assistant module; analyse the received input to identify one or more terms relating to a medical condition or a potential medical condition of the subject; obtain physiological characteristic and/or activity measurements for the subject from one or more physiological characteristic and/or activity sensors associated with the subject; process the identified one or more terms, medical history information for the subject and the obtained physiological characteristic and/or activity measurements to determine an adjustment to a measurement frequency and/or measurement sensitivity of the one or more physiological characteristic and/or activity sensors in order to obtain additional context information for the subject, the additional context information comprising one or more additional physiological characteristic and/or activity measurements for the subject; adjust the measurement frequency and/or measurement sensitivity of the one or more physiological characteristic and/or activity sensors according to the determined adjustment and obtain the additional context information using the one or more physiological characteristic and/or activity sensors; determine if a notification to a care provider is required based on the identified one or more terms, the medical history information, the obtained physiological characteristic and/or activity measurements and the obtained additional context information; generate, if a notification to a care provider is required, a summary for the care provider including information extracted from the conversation, the physiological characteristic and/or activity measurements and the additional context information; and cause the presentation of the generated summary to the care provider. Thus the invention improves the collection of data for a patient by adjusting the operation of the sensors, and improves the timing of the presentation of that data to a healthcare professional/care provider so that the healthcare professional can make the appropriate decision for the patient.

In some embodiments, the one or more terms comprise one or more symptoms and/or medications.

In some embodiments, the physiological characteristic and/or activity measurements comprise any one or more of: heart-related parameters, breathing-related parameters, blood pressure, body temperature, physical activity level, physical activity type and posture.

In some embodiments, the one or more processing units are configured to determine the adjustment to the measurement frequency and/or measurement sensitivity of the one or more physiological characteristic and/or activity sensors based on (i) physiological characteristic and/or activity measurements collected with respect to one or more previous conversations in which the one or more terms were also identified, and/or (ii) physiological characteristic and/or activity measurements required according to a protocol relating to the one or more identified terms. These embodiments provide for an appropriate adjustment to the sensors based on either or both of previous experience with the subject or other subjects and relevant protocols for those terms.

In some embodiments, the additional context information further comprises replies to one or more questions, and wherein the one or more processing units are further configured to: determine the one or more questions and/or instructions to the subject based on the identified one or more terms, the medical history information and the obtained physiological characteristic and/or activity measurements; provide the one or more questions and/or instructions to the virtual assistant module for presentation to the subject as part of the conversation; and receive, from the virtual assistant module, further input from the subject in response to the one or more questions and/or instructions.

In these embodiments, the one or more processing units can be further configured to: determine the one or more questions and/or instructions based on (i) information collected with respect to one or more previous conversations in which the one or more terms were also identified, (ii) information required according to a protocol relating to the one or more identified terms; and/or (iii) a predetermined dialogue flow relating to the one or more identified terms, wherein the dialogue flow comprises one or more predetermined questions and/or instructions. These embodiments provide for an appropriate interaction with the subject based on any or all of previous experience with the subject or other subjects, relevant protocols or dialogue flows for those terms.

In some embodiments, the one or more processing units are configured to determine the one or more questions or instructions from questions and/or instructions included in the one or more previous conversations and/or in the predetermined dialogue flow that have not yet been included in the conversation with the subject. In this way, the repetitiveness of the conversation with the subject should be minimised.

In some embodiments, the protocol indicates one or more of: one or more physiological characteristic and/or activity measurements to measure, one or more physiological characteristic and/or activity measurements to include in a generated summary, one or more questions to include in the conversation with the subject, and one or more instructions for the subject relating to actions the subject is to perform.

In some embodiments, the one or more processing units are further configured to: generate, if it is determined that a notification to the care provider is not required, on completion of the conversation, a summary for the care provider including information extracted from the conversation, the physiological characteristic and/or activity measurements and the additional context information; and cause the presentation of the generated summary to the care provider. In this way, the care provider is always provided with a summary of conversations in which a term relating to a medical condition or potential medical condition is provided by the subject.

In some embodiments, the one or more processing units are configured to generate the summary according to a predetermined template.

In some embodiments, the one or more processing units are configured to generate the summary to include one or more graphs and/or charts relating to the obtained physiological characteristic and/or activity measurements and any additional physiological characteristic and/or activity measurements. This can enable the care provider to more quickly assess the available information and take a decision on the subject.

In some embodiments, the one or more processing units are further configured to determine a priority value for the subject based on the identified one or more terms, the medical history information, the obtained physiological characteristic and/or activity measurements and any obtained additional context information, and to include the priority value in the generated summary. In this way the care provider or the care provider device can identify the subjects having the most urgent need for assistance or review.

In some embodiments, the one or more processing units are configured to cause the presentation of generated summaries for a plurality of subjects to the care provider and to order the presentation of the generated summaries according to respective priority values. In this way the care provider or the care provider device can identify the subjects having the most urgent need for assistance or review.

In some embodiments, the one or more processing units are configured to determine if notification to the care provider is required during the conversation with the subject, and, if notification is required, to generate the summary and cause the presentation of the generated summary to the care provider during the conversation. This provides the advantage that a care provider can be notified as soon as the apparatus determines that a notification is required, and potentially intervene in or take over the conversation, and it is not necessary to wait until the conversation between the subject and the virtual assistant module has ended.

In some embodiments, the one or more processing units are further configured to receive feedback from the care provider on the summary and/or the conversation with the subject.

In these embodiments, the one or more processing units can be further configured to update a conversation database and/or conversation module based on the received feedback, wherein the conversation database and/or conversation module is used to evaluate received inputs by the subject and to generate replies to the subject as part of the conversation. In this way the apparatus and/or the virtual assistant module can improve the relevance or usefulness of the questions and replies provided to the subject, and thereby improve the relevance or usefulness of the inputs provided by the subject.

In these embodiments, the one or more processing units can be further configured to update one or more predetermined adjustments to measurement frequency and/or measurement sensitivity based on the received feedback, wherein the one or more processing units are configured to determine the adjustment to the measurement frequency and/or measurement sensitivity from the one or more predetermined adjustments. In this way the apparatus can obtain the information required to assess whether a notification is required more quickly in the future, i.e. by setting the most appropriate measurement frequency and/or sensitivity straight away.

According to a second specific aspect, there is provided a method of assessing the health of a subject, the method comprising: receiving an input from a subject as part of a conversation between the subject and a virtual assistant module; analysing the received input to identify one or more terms relating to a medical condition or a potential medical condition of the subject; obtaining physiological characteristic and/or activity measurements for the subject from one or more physiological characteristic and/or activity sensors associated with the subject; processing the identified one or more terms, medical history information for the subject and the obtained physiological characteristic and/or activity measurements to determine an adjustment to a measurement frequency and/or measurement sensitivity of the one or more physiological characteristic and/or activity sensors in order to obtain additional context information for the subject, the additional context information comprising one or more additional physiological characteristic and/or activity measurements for the subject; adjusting the measurement frequency and/or measurement sensitivity of the one or more physiological characteristic and/or activity sensors according to the determined adjustment and obtain the additional context information using the one or more physiological characteristic and/or activity sensors; determining if a notification to a care provider is required based on the identified one or more terms, the medical history information, the obtained physiological characteristic and/or activity measurements and the obtained additional context information; generating, if a notification to a care provider is required, a summary for the care provider including information extracted from the conversation, the physiological characteristic and/or activity measurements and the additional context information; and causing the presentation of the generated summary to the care provider. Thus the invention improves the collection of data for a patient by adjusting the operation of the sensors, and improves the timing of the presentation of that data to a healthcare professional/care provider so that the healthcare professional can make the appropriate decision for the patient.

In some embodiments, the one or more terms comprise one or more symptoms and/or medications.

In some embodiments, the physiological characteristic and/or activity measurements comprise any one or more of: heart-related parameters, breathing-related parameters, blood pressure, body temperature, physical activity level, physical activity type and posture.

In some embodiments, the method comprises determining the adjustment to the measurement frequency and/or measurement sensitivity of the one or more physiological characteristic and/or activity sensors based on (i) physiological characteristic and/or activity measurements collected with respect to one or more previous conversations in which the one or more terms were also identified, and/or (ii) physiological characteristic and/or activity measurements required according to a protocol relating to the one or more identified terms. These embodiments provide for an appropriate adjustment to the sensors based on either or both of previous experience with the subject or other subjects and relevant protocols for those terms.

In some embodiments, the additional context information further comprises replies to one or more questions, and wherein the method comprises determining the one or more questions and/or instructions to the subject based on the identified one or more terms, the medical history information and the obtained physiological characteristic and/or activity measurements; providing the one or more questions and/or instructions to the virtual assistant module for presentation to the subject as part of the conversation; and receiving, from the virtual assistant module, further input from the subject in response to the one or more questions and/or instructions.

In these embodiments, the method can further comprise determining the one or more questions and/or instructions based on (i) information collected with respect to one or more previous conversations in which the one or more terms were also identified, (ii) information required according to a protocol relating to the one or more identified terms; and/or (iii) a predetermined dialogue flow relating to the one or more identified terms, wherein the dialogue flow comprises one or more predetermined questions and/or instructions. These embodiments provide for an appropriate interaction with the subject based on any or all of previous experience with the subject or other subjects, relevant protocols or dialogue flows for those terms.

In some embodiments, the method comprises determining the one or more questions or instructions from questions and/or instructions included in the one or more previous conversations and/or in the predetermined dialogue flow that have not yet been included in the conversation with the subject. In this way, the repetitiveness of the conversation with the subject should be minimised.

In some embodiments, the protocol indicates one or more of: one or more physiological characteristic and/or activity measurements to measure, one or more physiological characteristic and/or activity measurements to include in a generated summary, one or more questions to include in the conversation with the subject, and one or more instructions for the subject relating to actions the subject is to perform.

In some embodiments, the method further comprises generating, if it is determined that a notification to the care provider is not required, on completion of the conversation, a summary for the care provider including information extracted from the conversation, the physiological characteristic and/or activity measurements and the additional context information; and causing the presentation of the generated summary to the care provider. In this way, the care provider is always provided with a summary of conversations in which a term relating to a medical condition or potential medical condition is provided by the subject.

In some embodiments, the method comprises generating the summary according to a predetermined template.

In some embodiments, the method comprises generating the summary to include one or more graphs and/or charts relating to the obtained physiological characteristic and/or activity measurements and any additional physiological characteristic and/or activity measurements. This can enable the care provider to more quickly assess the available information and take a decision on the subject.

In some embodiments, the method comprises determining a priority value for the subject based on the identified one or more terms, the medical history information, the obtained physiological characteristic and/or activity measurements and any obtained additional context information, and to including the priority value in the generated summary. In this way the care provider or the care provider device can identify the subjects having the most urgent need for assistance or review.

In some embodiments, the method comprises causing the presentation of generated summaries for a plurality of subjects to the care provider and ordering the presentation of the generated summaries according to respective priority values. In this way the care provider or the care provider device can identify the subjects having the most urgent need for assistance or review.

In some embodiments, the method comprises determines if notification to the care provider is required during the conversation with the subject, and, if notification is required, generating the summary and causing the presentation of the generated summary to the care provider during the conversation. This provides the advantage that a care provider can be notified as soon as the apparatus determines that a notification is required, and potentially intervene in or take over the conversation, and it is not necessary to wait until the conversation between the subject and the virtual assistant module has ended.

In some embodiments, the method further comprises receiving feedback from the care provider on the summary and/or the conversation with the subject.

In these embodiments, the method further comprises updating a conversation database and/or conversation module based on the received feedback, wherein the conversation database and/or conversation module is used to evaluate received inputs by the subject and to generate replies to the subject as part of the conversation. In this way the virtual assistant module can improve the relevance or usefulness of the questions and replies provided to the subject, and thereby improve the relevance or usefulness of the inputs provided by the subject.

In these embodiments, the method can further include updating one or more predetermined adjustments to measurement frequency and/or measurement sensitivity based on the received feedback, wherein the adjustment to the measurement frequency and/or measurement sensitivity is determined from the one or more predetermined adjustments. In this way information that is required to assess whether a notification is required can be obtained more quickly in the future, i.e. by setting the most appropriate measurement frequency and/or sensitivity straight away.

According to a third aspect, there is provided a computer program product comprising a computer readable medium having computer readable code embodied therein, the computer readable code being configured such that, on execution by a suitable computer or processor, the computer or processor is caused to perform the method according to the second aspect or any embodiment thereof.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a block diagram of an exemplary system in which embodiments of the invention can be implemented;
Fig. 2 is a flow chart showing an exemplary sequence of steps/events when a term relating to a medical condition or a potential medical condition is identified in an input from a subject;
Fig. 3 illustrates an exemplary summary that can be presented to a care provider on a user interface; and
Fig. 4 is a flow chart showing an exemplary general method of assessing the health of a subject.

### DETAILED DESCRIPTION OF EMBODIMENTS

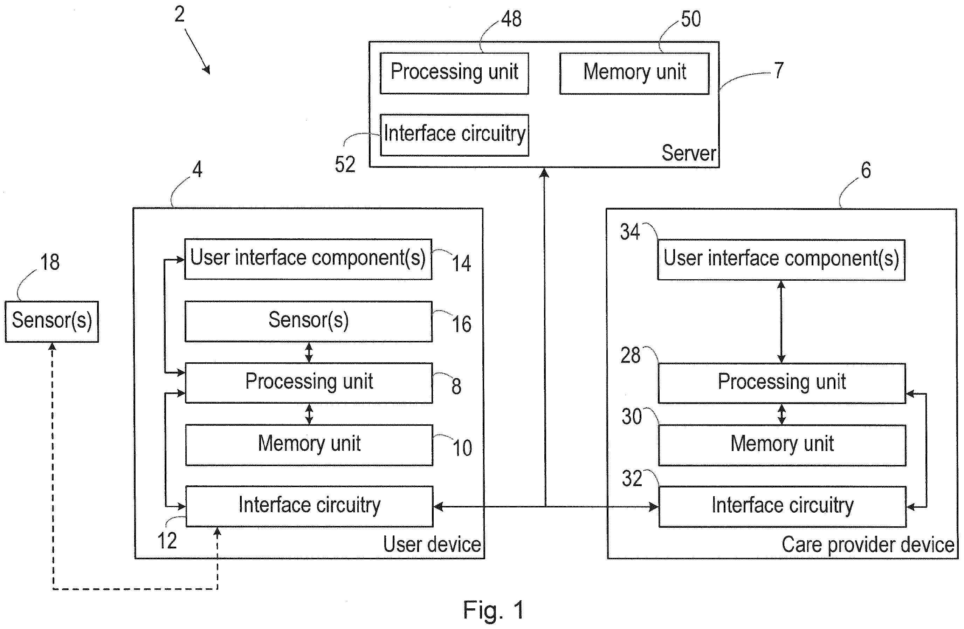

An exemplary system 2 in which embodiments of the invention can be implemented is shown. The system 2 comprises at least one user device 4 (only one user device 4 is shown in Fig. 1) that is to be used by person or patient (and who is referred to herein as the 'subject'), at least one care provider device 6 (only one care provider device 6 is shown in Fig. 1) that is to be used by a care provider, for example a physician or a nurse (who are also referred to herein as 'healthcare professionals'), and a server 7. The user device 4 can communicate with the care provider device 6 (and vice versa) either directly or via the server 7, using one or more wired and/or wireless communication links (including via the Internet). In some embodiments, the server 7 is not required (for example if the user device 4 and/or the care provider device 6 implement all of the steps of the techniques described herein), and thus the server 7 can be omitted.

In general, the user device 4 is for implementing a virtual assistant module that can engage the subject in a conversation and for obtaining measurements of a physiological characteristic and/or measurements of the activity (e.g. physical activity) of the subject. The user device 4 may be in the form of a smart phone, tablet, laptop computer, desktop computer, television, or any other type of consumer or domestic electronics device.

In general, the care provider device 6 is for providing information to a care provider about one or more subjects, for example information about symptoms, medication, recovery progress, etc. The care provider device 6 may be in the form of a smart phone, tablet, laptop computer, desktop computer, television, or any other type of consumer or professional electronics device.

The user device 4 includes a processing unit 8 that controls the operation of the user device 4 and that can be configured to execute or perform the methods, or part of the methods, described herein. The processing unit 8 can be implemented in numerous ways, with software and/or hardware, to perform the various functions described herein. The processing unit 8 may comprise one or more microprocessors or digital signal processor (DSPs) that may be programmed using software or computer program code to perform the required functions and/or to control components of the processing unit 8 to effect the required functions. The processing unit 8 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 8 is connected to a memory unit 10 that can store data, information and/or signals for use by the processing unit 8 in controlling the operation of the user device 4 and/or in executing or performing the methods, or part of the methods, described herein. In some implementations the memory unit 10 stores computer-readable code that can be executed by the processing unit 8 so that the processing unit 8 performs one or more functions, including the methods, or part of the methods, described herein. The memory unit 10 can comprise any type of non-transitory machine-readable medium, such as cache or system memory including volatile and non-volatile computer memory such as random access memory (RAM) static RAM (SRAM), dynamic RAM (DRAM), read-only memory (ROM), programmable ROM (PROM), erasable PROM (EPROM) and electrically erasable PROM (EEPROM), implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

The user device 4 also includes interface circuitry 12 for enabling a data connection to and/or data exchange with other devices, including any one or more of the care provider device 6, the server 7, databases, and sensors. The connection may be direct or indirect (e.g. via the Internet), and thus the interface circuitry 12 can enable a connection between the user device 4 and a network, such as the Internet, via any desirable wired or wireless communication protocol. For example, the interface circuitry 12 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). The interface circuitry 12 is connected to the processing unit 8.

The user device 4 also comprises user interface components 14 that include one or more components that enables the subject to input information (including inputs as part of a conversation with a virtual assistant module), data and/or commands into the user device 4, and/or enables the user device 4 to output information (including outputs as part of a conversation with a virtual assistant module), or data to the subject. The user interface components 14 can comprise any suitable input component(s), including but not limited to a keyboard, keypad, one or more buttons, switches or dials, a mouse, a track pad, a touchscreen, a stylus, a camera, a microphone, etc., and the user interface components 14 can comprise any suitable output component(s), including but not limited to a display screen, one or more lights or light elements, one or more loudspeakers, a vibrating element, etc.

In some embodiments, the user device 4 includes one or more internal sensors 16 for measuring one or more physiological characteristics of the subject or the physical activity of the subject. The one or more internal sensors 16 can be integral to the user device 4 and measure the physiological characteristics and/or activity when the subject is using the user device 4. In some embodiments (which can be in addition to or alternatively to the embodiments in which the user device 4 includes one or more internal sensors 16), the system 2 can comprise one or more external sensors 18 for measuring one or more physiological characteristics of the subject or the physical activity of the subject, and the one or more external sensors 18 are connected to the user device 4 (for example via the interface circuitry 12).

In either embodiment, the physiological characteristic can be, for example, heart rate, heart rate variability, blood pressure, breathing rate, breathing depth, skin temperature, skin conductivity, core body temperature, etc. The measurement of the physical activity can be an indication of the current physical activity of the subject (e.g. running, walking, sitting down, lying down, etc.), the posture of the subject (e.g. sitting, standing, lying on their back, etc.) or a general measure of activity level (e.g. number of steps in a time period, amount of time where activity level was above a threshold, etc.). As such, the sensors 16, 18 can include any type of sensor for measuring these physiological characteristics and/or physical activity measures. For example the sensors 16, 18 can be any of an accelerometer or other type of movement sensor, a heart rate monitor, an electrocardiography (ECG), a blood pressure monitor, a plethysmography (PG) sensor, a photoplethsmography (PPG) sensor, skin conductivity sensor, thermometer, a camera, etc.

It will be appreciated that a practical implementation of a user device 4 may include additional components to those shown in Fig. 1. For example the user device 4 may also include a power supply, such as a battery, or components for enabling the user device 4 to be connected to a mains power supply.

The care provider device 6 includes a processing unit 28 that controls the operation of the care provider device 6 and that can be configured to execute or perform the methods, or part of the methods, described herein. The processing unit 28 can be implemented in numerous different ways, similar to the processing unit 8 in the user device 4.

The processing unit 28 is connected to a memory unit 30 that can store data, information and/or signals for use by the processing unit 28 in controlling the operation of the care provider device 6 and/or in executing or performing the methods, or part of the methods, described herein. The memory unit 30 can be implemented in numerous different ways, similar to the memory unit 10 in the user device 4.

The care provider device 6 also includes interface circuitry 32 for enabling a data connection to and/or data exchange with other devices, including any one or more of the user device 4, the server 7, databases, and sensors. The connection may be direct or indirect (e.g. via the Internet), and thus the interface circuitry 32 can enable a connection between the care provider device 6 and a network, such as the Internet, via any desirable wired or wireless communication protocol. For example, the interface circuitry 32 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). The interface circuitry 32 is connected to the processing unit 28.

The care provider device 6 also comprises user interface components 34 that include one or more components that enables the care provider to input information, data and/or commands into the care provider device 6, and/or enables the care provider device 6 to output information (including a summary of information about a subject), or data to the care provider. The user interface components 34 can comprise any suitable input component(s), including but not limited to a keyboard, keypad, one or more buttons, switches or dials, a mouse, a track pad, a touchscreen, a stylus, a camera, a microphone, etc., and the user interface components 34 can comprise any suitable output component(s), including but not limited to a display screen, one or more lights or light elements, one or more loudspeakers, a vibrating element, etc.

It will be appreciated that a practical implementation of a care provider device 6 may include additional components to those shown in Fig. 1. For example the care provider device 6 may also include a power supply, such as a battery, or components for enabling the care provider device 6 to be connected to a mains power supply.

The server 7 includes a processing unit 48 that controls the operation of the server 7 and that can be configured to execute or perform the methods, or part of the methods, described herein. The processing unit 48 can be implemented in numerous different ways, similar to the processing unit 8 in the user device 4 and processing unit 28 in the care provider device 6.

The processing unit 48 is connected to a memory unit 50 that can store data, information and/or signals for use by the processing unit 48 in controlling the operation of the server 7 and/or in executing or performing the methods, or part of the methods, described herein. The memory unit 50 can be implemented in numerous different ways, similar to the memory unit 10 in the user device 4 and memory unit 30 in the care provider device 6.

The server 7 also includes interface circuitry 52 for enabling a data connection to and/or data exchange with other devices, including any one or more of the user device 4, the care provider device 6, databases, and sensors. The connection may be direct or indirect (e.g. via the Internet), and thus the interface circuitry 52 can enable a connection between the server 7 and a network, such as the Internet, via any desirable wired or wireless communication protocol. For example, the interface circuitry 52 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). The interface circuitry 52 is connected to the processing unit 48.

It will be appreciated that a practical implementation of a server 7 may include additional components to those shown in Fig. 1. For example the server 7 may also include user interface components similar to the user interface components 14 in the user device 4 or the user interface components 34 in the care provider device 6 to enable a user of the server to input information, data and/or commands into the server 7, and/or enable the server 7 to output information or data to a user of the server 7. The server 7 may also include a power supply, such as a battery, or components for enabling the server 7 to be connected to a mains power supply.

It will be appreciated from the explanation below that various parts or steps of the techniques described herein can be performed by any one or more of the processing units 8, 28, 48. In particular, in some implementations, each of the parts or steps of the techniques described herein can be performed by the processing unit 8 in the user device 4. In some other implementations, each of the parts or steps of the techniques described herein can be performed by the processing unit 28 in the care provider device 6. In some other implementations, each of the parts or steps of the techniques described herein can be performed by the processing unit 48 in the server 7. In yet other implementations, the techniques described herein can be implemented across any combination of two or more of the processing unit 8 in the user device 4, the processing unit 28 in the care provider device 6 and the processing unit 48 in the server 7, with suitable communications between the user device 4, the care provider device 6 and the server 7 to enable each other processing unit 8, 28, 48 (as appropriate) to perform the next part or step of the described techniques. In the following explanation, the invention will be described for simplicity as being implemented by an apparatus that includes one or more processing units 8, 28, 48, and it will be appreciated that the apparatus can correspond to any of the user device 4, the care provider device 6 and the server 7, or any combination thereof (where parts of the techniques described herein are implemented by different ones of the user device 4, the care provider device 6 and/or the server 7).

As noted above, there is a problem with keeping a care provider sufficiently informed about the status of their post-discharge patients ('subjects') and prioritizing the patients most in need, whilst keeping the patient engaged and effective in communicating their needs to the care provider. Therefore, the techniques described herein provide improvements in the collection of data for a subject to enable interpretation of symptoms, and improvements in the presentation of that data to a care provider so that the care provider can make the appropriate decisions for the subject. The techniques described herein also improve the speed and accuracy with which symptoms reported by subjects are diagnosed.

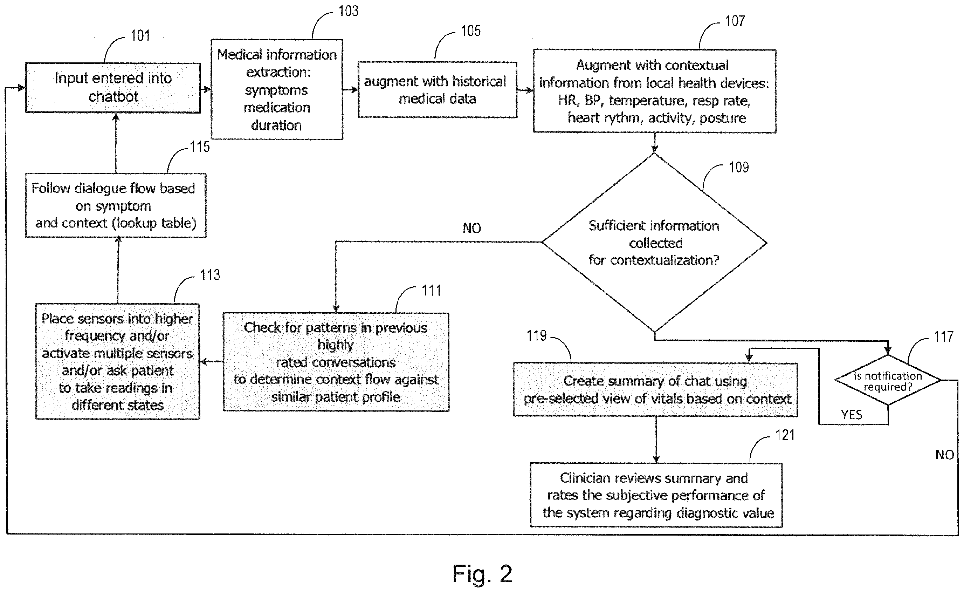

Thus, the user device 4 can provide a chat bot/virtual assistant interface to the subject that acts as an intermediary between the subject and the care provider when the care provider is not available or is not yet required to be available (e.g. because the subject has not yet reported any new symptoms or change in symptoms, or an interaction between the care provider and subject is not scheduled). In addition to analysing inputs provided by the subject as part of a conversation with the virtual assistant module (that provides the chat bot/virtual assistant interface) to determine a suitable response, the inputs can be analysed to identify particular keywords or trigger words from the subject (e.g. terms relating to a medical condition or a potential medical condition of the subject). An exemplary sequence of steps/events that can occur in system 2 when a term relating to a medical condition or a potential medical condition is identified in an input from the subject is set out in Fig. 2. It will be appreciated that each step or event shown in Fig. 2 can be implemented by the processing unit 8 in the user device 4, the processing unit 28 in the care provider device 6, the processing unit 48 in the server 7, or any combination thereof.

In step 101 an input is received from the subject as part of the conversation with the virtual assistant (chat bot) module. The input can be a spoken input (e.g. received using a microphone in the user device 4), a text-based input (e.g. received using a physical keyboard or touch-screen keyboard on the user device 4), or an input in any other suitable form.

In step 103 the input is analysed to extract medical information, such as symptoms, medication and duration. Step 103 can be performed by a natural language processing module implemented by any of the processing units 8, 28, 48, and it can determine any symptoms, the time of occurrence and duration.

In step 105 the medical information extracted from the input is augmented with historical medical data or information for the subject. This medical information can be retrieved from a medical history database that stores medical history information for the subject, and optionally a plurality of other subjects. In the former case the medical information may be stored in the memory unit 10 in the user device 4, in the memory unit 30 in the care provider device 6 or the memory unit 50 in the server 7. In the latter case (i.e. where the database stores medical information for a plurality of subjects), the medical information may be stored in the memory unit 30 in the care provider device 6 or the memory unit 50 in the server 7. The historical medical data or information can include trend data (e.g. for a physiological characteristic and/or physical activity) and/or medical records.

In step 107 the medical information extracted from the input and the medical history information is further augmented with contextual information about the subject obtained from the one or more sensors 16, 18 associated with the subject. The contextual information can include measurements of one or more physiological characteristics and/or measurements of the physical activity of the subject, and may be information that enables the medical information input by the subject to be placed into a suitable context. The contextual information may include current measurements of the physiological characteristics and/or physical activity and/or recent measurements of the physiological characteristics and/or physical activity (e.g. measurements obtained just before the input from the subject). As an example, the contextual information can include any of heart rate (HR), blood pressure (BP), temperatures, breathing (respiratory) rate, heart rhythm, activity and posture. Step 107 can be performed by a context processing module implemented by any of the processing units 8, 28, 48. The context processing module can therefore determine the contextual information required to put the medical information extracted from the input into context and to obtain that information in step 107. Some exemplary types of context information to be obtained for particular medical information keywords are provided below in Table 1.

In step 109, it is determined whether sufficient information has been collected for contextualisation of the medical information extracted from the subject's input (i.e. can the extracted medical information be put into context). If sufficient information is available then it is possible (in a subsequent step) to determine whether the subject needs to be escalated to the care provider, or even to an emergency service (e.g. an ambulance). It can be determined whether sufficient information has been collected based on standard clinical protocols, and/or previous interactions/conversations with the subject or a population of subjects.

If it is determined that sufficient information has not yet been collected, then in step 111 previous conversations (in some embodiments conversations that were rated highly by a care provider) with either the subject or other subjects are evaluated to identify patterns in those conversations corresponding to the medical information extracted in step 103, the medical history data obtained in step 105 and/or the contextual information obtained in step 107. These patterns can indicate how the context information should be augmented based on the profile of the subject to provide sufficient information for contextualising the extracted medical information.

For example, if the symptom reported by the subject in their conversation input is recent (i.e. occurred recently), step 111 can comprise determining that the measurement frequency for one or more of the sensors 16, 18 should be increased in order to gain a more accurate/more recent measurement of a relevant physiological characteristic. As another example, step 111 can comprise determining a number of actions that the subject is to be requested to perform, such as taking a blood pressure measurement, or taking a nitroglycerin (NTG) spray. Step 111 may also determine that the conversation (or that particular topic of conversation) should be resumed with the subject after a particular period of time (some examples of this are provided in Table 1 below). In a particular example, if the subject mentions in the conversation that they are experiencing palpitations, then step 111 can comprise determining that the heart rate and/or ECG signal should be measured more frequently (or at a maximum frequency) so that this information can subsequently be presented to the care provider in a summary so that they can determine whether the subject has arrhythmia.

Following step 113, additional context information can be obtained using the one or more sensors 16, 18. Step 113 can comprise using the one or more sensors 16, 18 to obtain physiological characteristic and/or activity measurements more frequently than previously (i.e. measurements are obtained with a higher sampling frequency, e.g. every few seconds rather than once per hour), activating one or more sensors 16, 18 that have not yet been used to measure a physiological characteristic and/or physical activity (i.e. measuring blood pressure following previous measurements of heart rate), and/or adjusting the sensitivity of the one or more sensors 16, 18 to obtain measurements with an increased accuracy/reduced error (which may include providing instructions to the subject to perform or stop a particular activity, e.g. sit down and rest so a blood pressure measurement can be obtained).

In step 115, a dialogue flow (e.g. particular questions and/or instructions for the subject) can be initiated based on the extracted medical information (i.e. the extracted symptoms) and/or the patterns identified in step 111. Steps 113 and 115 provide further information to enable the severity of the extracted symptom to be evaluated and to cross-correlate the extracted symptom with other factors such as physical activity level, respiration rate, heart rate variability, arrhythmia signs, blood pressure, and posture.

In an initial state, a dialogue flow with the subject provided by the virtual assistant module can be based on standard clinical protocols (e.g. a chest pain evaluation protocol if chest pain is reported by the subject). Over time, dialogue flows can be updated and refined based on outputs that are more successful and/or rated more highly by the recipients (i.e. the care provider) and/or by the subjects involved.

After step 115 the method returns to step 101 to evaluate further responses by the subject and subsequently determines whether sufficient context information has been collected for contextualisation (step 109). Alternatively after step 115 the method can return directly to step 109.

If at step 109 it is determined that sufficient context information has been collected for contextualisation, then it can be determined whether a notification to a care provider is required (step 117). A notification to a care provider can be required if the method determines that the subject is in need of medical assistance or a medical consultation with the care provider.

If it is determined that a notification is not required (for example if the context information indicates that a subject's reported symptom of light headedness was due to overly strenuous exercise and the subject is currently okay), then the method can return to step 101.

If it is determined that a notification is required, a summary of the conversation and context information (e.g. the measurements of the physiological characteristics and/or physical activity and/or medical history information) is generated for the care provider (step 119). The summary can be generated according to a template for the particular symptom reported by the subject, according to a template selected for a particular subject, according to a template selected for a particular care provider or according to the type(s) of information available for the subject. The summary should enable the care provider to quickly assimilate all of the important information about the subject and make a decision about whether contact is required with the subject or not. Table 1 below indicates the type of information that can be provided to a care provider for particular reported symptoms. In some embodiments, step 119 can include generating a priority score or priority value (e.g. high, medium or low priority) for the subject indicating how important it is for the summary about the subject to be reviewed quickly by the care provider.

In step 121 the summary is presented to the care provider via the user interface components 34 in the care provider device 6. The care provider reviews the summary and makes a decision. At this stage the care provider may be able to take over the conversation with the subject, or separately engage the subject, for example by making a telephone or video call.

Also in step 121, the care provider may provide feedback to the method regarding the generated summary, for example based on how useful the summary is in terms of content and/or accuracy (e.g. in diagnosing a problem in the subject), and/or based on the content dialogue flow with the subject. A machine learning algorithm, e.g. a deep learning net, can be used to train or improve dialogue flows, measurement frequencies/sensitivities and generated summaries based on this feedback. For example the feedback can be used to teach the system which dialogue flows, sensor states and summaries are most appropriate for a given subject profile.

Table 1 below indicates various different symptom keywords that can be identified in inputs by a subject, the type of information to be included in a summary for the care provider, dialogue questions to the subject and any subsequent actions the subject is to perform and subsequent monitoring triggers (e.g. new measurements to take before determining whether a summary is required).

**Table 1**

| **Symptoms key words** | **Graphs to auto-present to care provider** | **Dialogue Questions to subject and subsequent actions** | **Subsequent monitoring triggers** |
|---|---|---|---|
| Dizzy, Light headed, woozy | Blood pressure, Activity level | Check activity level is low for 10 minutes and ask to take blood pressure reading now. Check that activity is low prior to reading. | Blood pressure Posture (BP laying down vs sitting vs standing) Activity level |
| Sweating | Heart rate, activity | Answer questions about what they were doing and how strenuous it was. What is the temperature? Is there a pain in the back or jaw? | Activity level |
| Chest pain, pain in chest | Heart rate, symptom history, activity | Show scale in app. Take NTG spray and respond how they feel after 5 minutes Stay inactive | Raw PPG signal analysis. Send alert to care provider |
| Breathless, out of breath, short of breath | Activity, Heart rate, Respiration rate, | Answer questions about what they were doing that led to the breathlessness | Raw PPG signal analysis |
| Thudding, palpitations, irregular heart beat | Heart rate, Inter-beat Blood volume pulse | ... | Raw PPG signal analysis. Detect of arrhythmia |
| Fatigue, tired, slow, heavy | Activity level, heart rate | ... | ... |

An exemplary dialogue flow between a subject and a virtual assistant (VA) is set out below.
Subject: I felt really dizzy this **morning** [here the method identifies 'dizzy' as a medical keyword and 'morning' as the timing of this symptom]
VA: I see that you were very active this morning. What were you doing?
Subject: I was just doing gardening, but think I overdid things.
VA: Let's take a few minutes of rest and take a blood pressure reading together. [some time elapses]
VA: You appear nice and rested, so it's time to take your blood pressure. [a blood pressure measurement is taken]
VA: It appears to be lower than your normal recent reading. Let's try again in an hour. If it is still low, let's inform the care provider.

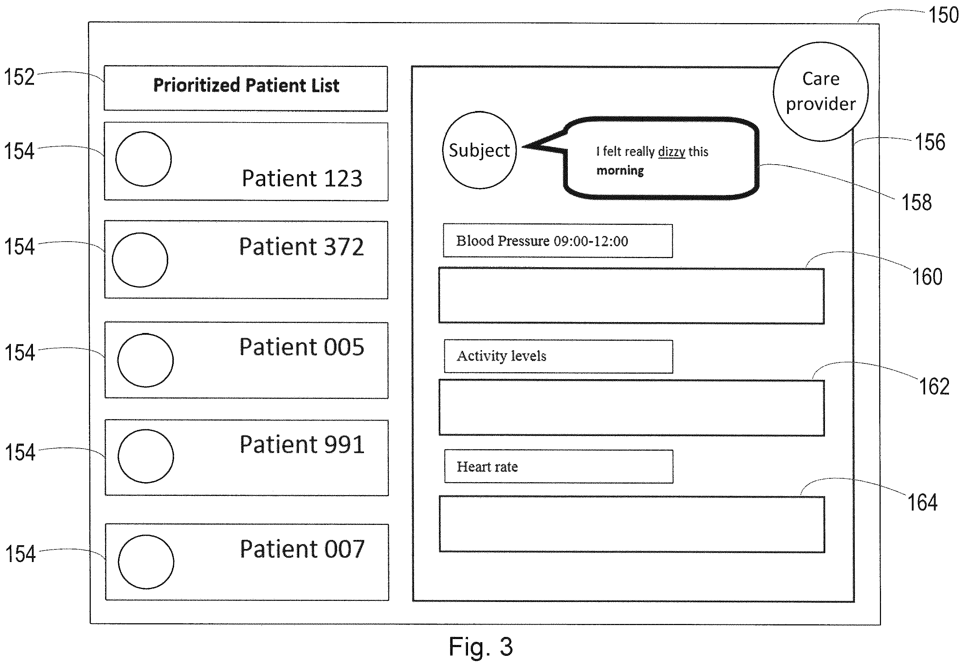

Fig. 3 illustrates an exemplary user interface that can be presented to the care provider when a respective summary is generated for one or more subjects. The user interface 150 includes a prioritized patient list 152 that lists the patients (subjects) for which summaries have been received. Five patients 154 are shown in the prioritized list 152, and the patients 154 are ordered according to a priority value associated with the patient. On selecting a patient from the list 152 (the patient 154 at the top of the priority list 152 in Fig. 3), the user interface 150 displays the summary 156 for that patient 154. The summary 156 includes an extract 158 from the conversation (in this case the part of the conversation that triggered the method in Fig. 2), a graph 160 showing blood pressure measurements for the patient in a 3-hour time period, a graph 162 showing activity levels of the patient over the 3-hour time period, and a graph 164 showing the heart rate of the patient over the 3-hour time period. Although not shown in Fig. 3, the user interface 150 can provide the care provider with an option to call the patient associated with the generated summary, or to intervene in and take over the conversation managed by the virtual assistant.

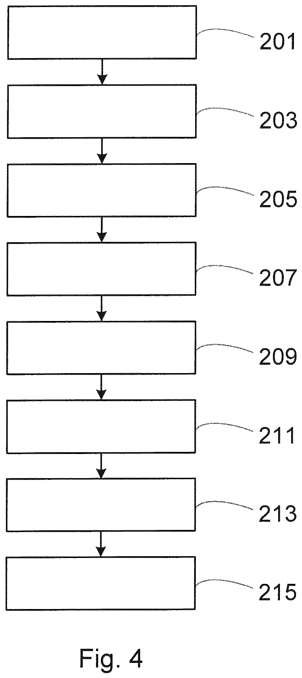

The flow chart in Fig. 4 shows an exemplary general method of assessing the health of a subject according to the techniques described herein. As noted above, the method can be performed by any combination of the processing unit 8 of the user device 4, the processing unit 28 of the care provider device 6 and the processing unit 48 of the server 7. Any of the processing units 8, 28, 48 can be caused to perform the required method step or steps by executing suitable computer program code stored in the respective memory unit 10, 30,50.

In a first step, step 201, an input is received from a subject as part of a conversation between the subject and a virtual assistant module. As noted above, the input can be a spoken input by the subject or a text-based input (e.g. via a physical or on-screen keyboard). The input could also or alternatively be in respect of a particular user interface element displayed or presented to the subject, such as a scale or a graphical element such as a widget. The input may be received in step 201 during a conversation with the virtual assistant, or as the opening input of a conversation with the virtual assistant.

In step 203, the received input is analysed to identify one or more terms relating to a medical condition or a potential medical condition of the subject. For example the one or more terms could be, or relate to, symptoms, potential symptoms and/or medications.

In step 205, physiological characteristic and/or activity measurements for the subject are obtained from one or more physiological characteristic and/or activity sensors 16, 18 associated with the subject (for example carried or worn by the subject). The physiological characteristic and/or activity measurements can comprise any one or more of heart-related parameters, breathing-related parameters, blood pressure, body temperature, physical activity level, physical activity type and posture.

In step 207, the identified one or more terms, medical history information for the subject and the obtained physiological characteristic and/or activity measurements are processed to determine an adjustment to a measurement frequency (e.g. sampling frequency) and/or measurement sensitivity of the one or more physiological characteristic and/or activity sensors 16, 18 in order to obtain additional context information for the subject. This step may also include identifying additional sensor(s) 16, 18 to use to measure the physiological characteristics and/or activity of the subject (which can be understood as modifying a sampling frequency for that particular sensor 16, 18 from zero to a non-zero value). The additional context information comprises one or more additional physiological characteristic and/or activity measurements for the subject. In some embodiments, the adjustment can be determined based on physiological characteristic and/or activity measurements collected with respect to one or more previous conversations (dialogues) in which the one or more terms were also identified. In addition or alternatively, the adjustment can be determined based on physiological characteristic and/or activity measurements required according to a protocol (e.g. a clinical protocol) relating to the one or more identified terms. The protocol may indicate one or more of: one or more physiological characteristic and/or activity measurements to measure, one or more physiological characteristic and/or activity measurements to include in a generated summary, one or more questions to include in the conversation with the subject, and one or more instructions for the subject relating to actions the subject is to perform.

In some embodiments, the additional context information further comprises replies to one or more questions. In this case, as part of step 207 or along side step 207, one or more questions and/or instructions to the subject can be determined based on the identified one or more terms, the medical history information and the obtained physiological characteristic and/or activity measurements. Some examples of additional questions and instructions are shown in Table 1.

The one or more questions and/or instructions can be determined based on information collected with respect to one or more previous conversations in which the one or more terms were also identified, information required according to a protocol (e.g. a clinical protocol) relating to the one or more identified terms, and/or a predetermined dialogue flow relating to the one or more identified terms. In addition the one or more questions or instructions can be determined by comparison to questions and/or instructions that have already been included in the conversation with the subject.

Then, along side step 209, the one or more questions and/or instructions are provided to the virtual assistant module for presentation to the subject as part of the conversation. The one or more questions and/or instructions are then provided to the subject. The virtual assistant module provides further input(s) from the subject in response to the one or more questions and/or instructions. Optionally these further inputs can be analysed according to step 203 above.

Next, in step 209, the measurement frequency and/or measurement sensitivity of the one or more physiological characteristic and/or activity sensors 16, 18 is adjusted according to the determined adjustment and the additional context information is obtained using the one or more physiological characteristic and/or activity sensors 16, 18. This step may comprise the processing unit 8 controlling the relevant sensors 16, 18 to operate at the required frequency and/or sensitivity.

Once the additional context information has been obtained, in step 211 it is determined if a notification to a care provider is required based on the identified one or more terms, the medical history information, the obtained physiological characteristic and/or activity measurements and the obtained additional context information.

If a notification to a care provider is required, then in step 213 a summary is generated for the care provider. The generated summary includes information extracted from the conversation, the physiological characteristic and/or activity measurements and the additional context information. The summary can be generated according to a predetermined template. The generated summary may include one or more graphs and/or charts relating to the obtained physiological characteristic and/or activity measurements and any additional physiological characteristic and/or activity measurements obtained in step 209.

In some embodiments, a priority value for the subject may also be determined based on the identified one or more terms, the medical history information, the obtained physiological characteristic and/or activity measurements and any obtained additional context information. The priority value can be included in, or otherwise associated with (e.g. included in a label or indicated in metadata) the generated summary.

Then, in step 215, the presentation of the generated summary to the care provider is triggered (for example by sending the generated summary (e.g. in the form of a data file or control signal) to the care provider device 6). In embodiments where a priority value is determined, the presentation of the generated summary can be in accordance with the determined priority value (i.e. other summaries having a higher priority value can be presented to the care provider first).

It will be appreciated that steps 203-215 can be performed while the conversation with the subject is ongoing, so in the most urgent or important cases (i.e. the subject indicates and exhibits symptoms that require immediate assistance) can be presented to the care provider as soon as possible.

In some embodiments, if it is determined in step 211 that a notification to the care provider is not required, then on completion of the conversation, a summary for the care provider can be generated including information extracted from the conversation, the physiological characteristic and/or activity measurements and the additional context information. The summary can be generated according to a predetermined template. This generated summary can then be sent to the care provider device 6 for presentation to the care provider. As above, a priority value can be determined for the generated summary. In this case, as a notification to the care provider is not required, the priority value can be low or a low value.

In some embodiments, feedback can be received from the care provider on the generated summary and/or the conversation with the subject. For example the feedback can indicate how useful the generated summary was in making a decision on the subject, how accurate the information contained in the generated summary was, how appropriate or useful the content of the conversation was, etc.

The feedback can be used to update a conversation database and/or the conversation module (i.e. the virtual assistant module) that uses the conversation database.

In some embodiments, in step 207 the adjustments to the measurement frequency and/or measurement sensitivity are determined from the one or more predetermined adjustments (e.g. predetermined for particular medical terms). In this case, the feedback can be used to update the one or more predetermined adjustments to measurement frequency and/or measurement sensitivity.

There are therefore provided techniques that improve the collection of data for a subject to enable interpretation of symptoms, and that improve the presentation of that data to a care provider so that the care provider can make the appropriate decisions for the subject.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (4, 6, 7) for assessing the health of a subject, the apparatus (4, 6, 7) comprising:
one or more processing units (8, 28, 48) configured to:
receive an input from a subject as part of a conversation between the subject and a virtual assistant module;
analyse the received input to identify one or more terms relating to a medical condition or a potential medical condition of the subject;
obtain physiological characteristic and/or activity measurements for the subject from one or more physiological characteristic and/or activity sensors (18) associated with the subject;
process the identified one or more terms, medical history information for the subject and the obtained physiological characteristic and/or activity measurements to determine an adjustment to a measurement frequency and/or measurement sensitivity of the one or more physiological characteristic and/or activity sensors (18) in order to obtain additional context information for the subject, the additional context information comprising one or more additional physiological characteristic and/or activity measurements for the subject;
adjust the measurement frequency and/or measurement sensitivity of the one or more physiological characteristic and/or activity sensors (18) according to the determined adjustment and obtain the additional context information using the one or more physiological characteristic and/or activity sensors (18);
determine if a notification to a care provider is required based on the identified one or more terms, the medical history information, the obtained physiological characteristic and/or activity measurements and the obtained additional context information;
generate, if a notification to a care provider is required, a summary for the care provider including information extracted from the conversation, the physiological characteristic and/or activity measurements and the additional context information; and
cause the presentation of the generated summary to the care provider.

2. An apparatus (4, 6, 7) as claimed in claim 1, wherein the one or more processing units (8, 28, 48) are configured to determine the adjustment to the measurement frequency and/or measurement sensitivity of the one or more physiological characteristic and/or activity sensors (18) based on (i) physiological characteristic and/or activity measurements collected with respect to one or more previous conversations in which the one or more terms were also identified, and/or (ii) physiological characteristic and/or activity measurements required according to a protocol relating to the one or more identified terms.

3. An apparatus (4, 6, 7) as claimed in claim 1 or 2, wherein the additional context information further comprises replies to one or more questions, and wherein the one or more processing units (8, 28, 48) are further configured to:
determine the one or more questions and/or instructions to the subject based on the identified one or more terms, the medical history information and the obtained physiological characteristic and/or activity measurements;
provide the one or more questions and/or instructions to the virtual assistant module for presentation to the subject as part of the conversation; and
receive, from the virtual assistant module, further input from the subject in response to the one or more questions and/or instructions.

4. An apparatus (4, 6, 7) as claimed in claim 3, wherein the one or more processing units (8, 28, 48) are further configured to:
determine the one or more questions and/or instructions based on (i) information collected with respect to one or more previous conversations in which the one or more terms were also identified, (ii) information required according to a protocol relating to the one or more identified terms; and/or (iii) a predetermined dialogue flow relating to the one or more identified terms, wherein the dialogue flow comprises one or more predetermined questions and/or instructions.

5. An apparatus (4, 6, 7) as claimed in claim 4, wherein the one or more processing units (8, 28, 48) are configured to determine the one or more questions or instructions from questions and/or instructions included in the one or more previous conversations and/or in the predetermined dialogue flow that have not yet been included in the conversation with the subject.

6. An apparatus (4, 6, 7) as claimed in any of claims 3, 4 or 5, wherein the protocol indicates one or more of: one or more physiological characteristic and/or activity measurements to measure, one or more physiological characteristic and/or activity measurements to include in a generated summary, one or more questions to include in the conversation with the subject, and one or more instructions for the subject relating to actions the subject is to perform.

7. An apparatus (4, 6, 7) as claimed in any of claims 1-6, wherein the one or more processing units (8, 28, 48) are further configured to:
generate, if it is determined that a notification to the care provider is not required, on completion of the conversation, a summary for the care provider including information extracted from the conversation, the physiological characteristic and/or activity measurements and the additional context information; and
cause the presentation of the generated summary to the care provider.

8. An apparatus (4, 6, 7) as claimed in any of claims 1-7, wherein the one or more processing units (8, 28, 48) are further configured to determine a priority value for the subject based on the identified one or more terms, the medical history information, the obtained physiological characteristic and/or activity measurements and any obtained additional context information, and to include the priority value in the generated summary.

9. An apparatus (4, 6, 7) as claimed in claim 8, wherein the one or more processing units (8, 28, 48) are configured to cause the presentation of generated summaries for a plurality of subjects to the care provider and to order the presentation of the generated summaries according to respective priority values.

10. An apparatus (4, 6, 7) as claimed in any of claims 1-9, wherein the one or more processing units (8, 28, 48) are configured to determine if notification to the care provider is required during the conversation with the subject, and, if notification is required, to generate the summary and cause the presentation of the generated summary to the care provider during the conversation.

11. An apparatus (4, 6, 7) as claimed in any of claims 1-10, wherein the one or more processing units (8, 28, 48) are further configured to receive feedback from the care provider on the summary and/or the conversation with the subject.

12. An apparatus (4, 6, 7) as claimed in claim 11, wherein the one or more processing units (8, 28, 48) are further configured to update a conversation database and/or conversation module based on the received feedback, wherein the conversation database and/or conversation module is used to evaluate received inputs by the subject and to generate replies to the subject as part of the conversation.

13. An apparatus (4, 6, 7) as claimed in claim 11 or 12, wherein the one or more processing units (8, 28, 48) are further configured to update one or more predetermined adjustments to measurement frequency and/or measurement sensitivity based on the received feedback, wherein the one or more processing units (8, 28, 48) are configured to determine the adjustment to the measurement frequency and/or measurement sensitivity from the one or more predetermined adjustments.

14. A method of assessing the health of a subject, the method comprising:
receiving (201) an input from a subject as part of a conversation between the subject and a virtual assistant module;
analysing (203) the received input to identify one or more terms relating to a medical condition or a potential medical condition of the subject;
obtaining (205) physiological characteristic and/or activity measurements for the subject from one or more physiological characteristic and/or activity sensors associated with the subject;
processing (207) the identified one or more terms, medical history information for the subject and the obtained physiological characteristic and/or activity measurements to determine an adjustment to a measurement frequency and/or measurement sensitivity of the one or more physiological characteristic and/or activity sensors in order to obtain additional context information for the subject, the additional context information comprising one or more additional physiological characteristic and/or activity measurements for the subject;
adjusting (209) the measurement frequency and/or measurement sensitivity of the one or more physiological characteristic and/or activity sensors according to the determined adjustment and obtain the additional context information using the one or more physiological characteristic and/or activity sensors;
determining (211) if a notification to a care provider is required based on the identified one or more terms, the medical history information, the obtained physiological characteristic and/or activity measurements and the obtained additional context information;
generating (213), if a notification to a care provider is required, a summary for the care provider including information extracted from the conversation, the physiological characteristic and/or activity measurements and the additional context information; and
causing (215) the presentation of the generated summary to the care provider.

15. A computer program product comprising a computer readable medium having computer readable code embodied therein, the computer readable code being configured such that, on execution by a suitable computer or processor, the computer or processor is caused to perform the method according to claim 14.
